# EUROPEAN PATENT SPECIFICATION

(11) **EP 3 427 717 B1**
(45) Date of publication and mention of the grant of the patent: **27.05.2020**
(21) Application number: 17840525.4
(22) Date of filing: 18.07.2017
(51) Int. Cl.: A61K 8/02, A61Q 19/08, A61Q 19/00, A61K 8/67, A61K 8/73, A61K 8/64, A61K 8/65, A61K 8/9794, A61K 8/9789, A61K 8/60, A61K 8/81, A61K 8/34, A61K 8/365, A61K 8/49

(54) **CONSTRUCTED GEL-FILLED COSMETIC MASK**
KONSTRUIERTE GELGEFÜLLTE KOSMETISCHE MASKE
MASQUE COSMÉTIQUE REMPLI DE GEL, CONSTRUIT

(30) Priority: 15.05.2017 CN 201710338291
(43) Date of publication of application: 16.01.2019
(73) Proprietor: Ruan, Shixing, Renhe Town, Baiyun District Guzangzhou City Guangdong 510000 (CN)
(72) Inventor: Ruan, Shixing, Renhe Town, Baiyun District Guzangzhou City Guangdong 510000 (CN)
(74) Representative: Laufhütte, Dieter
(86) International application number: PCT/CN2017/093409
(87) International publication number: WO 2018/209789

(56) References cited:
- EP-A1- 0 309 309
- WO-A1-2017/034215
- CN-A- 104 434 544
- CN-A- 105 476 867
- CN-Y- 201 350 283
- KR-A- 20140 064 139
- US-A1- 2002 022 884
- US-A1- 2006 286 152
- US-A1- 2009 305 024

## Description

### Technical Field

The present invention relates to a structured gel-filled cosmetic mask, and more particularly to a gel-filled cosmetic mask that is more suitable for use due to its structural stability and good stretchability.

### Background

At present, the cosmetic masks (such as facial masks, eye masks, etc.) in the market can be roughly divided into three types. The first type is made of non-woven or cloth, silk, fibers and so on in a shape similar to human face or eye peripheral area, which is then immersed in a solution containing active ingredients, and it is directly attached to face when using. The second type is inelastic fast-drying jelly type mask, such jelly mask or eye mask usually is placed in a sealed plastic box or bag. The sealed plastic box or bag is torn and the mask is attached when using. The third type is cataplasm mask, which is made by coating or bonding a cataplasm on a non-woven and cutting into a shape for use. Masks comprising intertwined and interlinked electrospun fibers are known from WO2017034215.

The shortcomings of the masks made of non-woven or cloth, silk, fiber and so on mainly include lack of elasticity and lack of continuous effects to facial skin, since their active ingredients are directly exposed to air and would be volatile or dry in a short period of time, so that they cannot act on the skin surface for a long time to achieve the cosmetic purpose in high efficiency. In addition, the masks made of non-woven or cloth, silk, fiber and so on initially contain a large amount of water, which makes skin absorbs excessive water at the beginning, and then the fast-dried fiber or cloth would absorb moisture from skin in reverse. This causes the skin to experience too much water replenishment and dryness in a short period of time, and the skin would be flabby more quickly after long-term use of such masks.

The disadvantages of jelly masks include insufficient elasticity and poor tenacity, as well as containing a lot of water, ease of volatilization and drying, ease of sliding and shifting on skin, ease of tearing when stretched after being affixed to face, insufficient stretchability and flexibility, unable to be firmly attached to skin, prone to be dry after contacting with air leading to quick volatilization of active ingredients, so that they cannot bring moisturizing and cosmetic effects for a long period of time .

The cataplasm type masks have skin irritation, likely to cause skin allergies, and are insoluble in water, airtight and cannot be used for a long time on the skin. In addition, cataplasm is insoluble in water after forming, resulting in the active ingredients cannot be transdermally absorbed. Further, since human face has three-dimensional contour and is unlike a plane, the cataplasm type masks have no stretchability and elastic conformability, would form wrinkles and upwarp during adhesion and not be able to fully fit the face.

At present, the use of facial masks is a simple and easy-to-use cosmetic method for most people. However, how to make external facial masks more effective and longer lasting, also to solve the problems of the current products, most importantly, to remove wrinkles on face and around eye and to provide continuity of cosmetic effects are the issues that need to be addressed.

### Contents of the Invention

The present invention aims to provide a structured gel-filled cosmetic mask, in which architectural structure and method are incorporated into cosmetic mask, employing a fixing skeleton, a gel filling band and a free-flow gel layer in an integrally formed structure that utilizes the cavity of concave structure of the fixing skeleton, in combination with the cosmetic ingredients in gel form, to produce elasticity and stretching force, make the cosmetic mask more stable, bearing more active ingredients, and exhibiting stretch effect when applying on skin wrinkles.

The technical solution adopted by the present invention is as follows:
A structured gel-filled cosmetic mask, comprising a fixing skeleton, a gel filling band and a free-flow gel layer in an integrally formed structure, wherein the fixing skeleton comprises a cavity of a concave structure, the cavity is filled with a premade filling material to form the gel filling band, and the gel in the gel filling band extends outward to form the free-flow gel layer.

Preferably, the cavity of the fixing skeleton is triangular, square, polygonal or irregular in shape, and a plurality of cavities are arranged in an array.

More preferably, the shape of the cavity of the fixing skeleton is a honeycomb shape or a regular hexagon, and a plurality of cavities are arranged in an array.

Further, a single cavity of the fixing skeleton has an area of no more than 9 mm².

Further, the cavity of the fixing skeleton has a diameter of 0.09 mm to 1.0 mm, and is preferably made of polyester fiber.

Further, the gel in the gel-filled band has a viscosity of 150,000 to 500,000 mPa·s and a density of 0.5 to 1.5 g/cm³.

Preferably, the free-flow gel layer coats both sides of the gel-filled band, and the thickness ratio of the gel-filled band to the free-flow gel layer is 1:2 to 1:10.

Preferably, the premade filling material is selected from the following four formulas:

**Formula 1:**

| | |
|---|---|
| Glycerol | 25% |
| Water | 66.05% |
| Sodium Polyacrylate NOVETAC P46N | 5.0% |
| Sodium Polyacrylate NOVETAC P55N | 0.5% |
| Dihydroxyaluminum aminoacetate | 0.15% |
| Hydrolyzed elastin | 1.0% |
| Sodium Hyaluronate | 0.5% |
| Vitamin E | 0.5% |
| VC polysaccharide | 0.3% |
| Paraben | 0.1% |
| Tartaric acid | 0.2% |
| Essence | 0.2% |
| Ethanol | 0.5% |

**Formula 2**

| | |
|---|---|
| Glycerin | 23% |
| Water | 65.34% |
| Sodium Polyacrylate AP800 | 6.0% |
| Dihydroxyaluminum aminoacetate | 0.16% |
| Sodium Hyaluronate | 0.5% |
| Collagen | 1.0% |
| Aloe leaf extract | 1.0% |
| Arbutin | 0.5% |
| Vitamin C | 0.3% |
| Hydantoin DMDM | 0.2% |
| Tartaric acid | 0.2% |
| Essence | 0.2% |
| Ethanol | 0.6% |

**Formula 3**

| | |
|---|---|
| Water | 46.15% |
| Glycerin | 44.9% |
| Carboxymethyl cellulose | 3.0% |
| Lubrajel | 0.8% |
| Xanthan gum | 1.0% |
| Pectin | 1.0% |
| Disodium EDTA | 0.5% |
| Sodium Hyaluronate | 1.0% |
| Aloe vera Leaf Extract | 1.0% |
| Aloperine | 0.3% |
| Oat peptide | 0.2% |
| Peppermint | 0.05% |
| Essence | 0.1% |

**Formula 4**

| | |
|---|---|
| Water | 24% |
| Glycerin | 64.5% |
| Hydroxyethyl cellulose | 3.0% |
| Konjac gum | 3.0% |
| Carbopol resin | 0.8% |
| Disodium EDTA | 0.5% |
| Arbutin | 2.0% |
| Sodium Hyaluronate | 0.5% |
| Egg White-Sodium | 0.1% |
| Collagen | 1.0% |
| Essence | 0.1% |
| Sodium hydroxide | 0.5% |

The structured gel-filled cosmetic mask according to the technical solution of the present invention is prepared by the following steps:
Step 1), preparation of the premade filling material: weighing ingredients, loading the ingredients into a mixer, mixing and emulsifying to form the premade filing material;
Step 2), injection: heating the premade filing material, and pouring or pumping the premade filling material into hoppers on both sides of a special coater; passing the fixing skeleton through two middle coating wheels between the hoppers on both sides; starting the coating wheels to rotate, injecting the gel downflowing from the hoppers on both sides into the fixing skeleton by the coating wheels to form an integrally formed matrix membrane;
Step 3), formation of mask: conveying the integrally formed matrix membrane as prepared in Step 2) via a conveying wheel to a gel curing channel, accelerating the curing of the matrix membrane in the curing channel by a high temperature drying method or a cooling method, then conveying to a die-cutting platform, selecting a die according to the shape of the product to be produced, and cutting the membrane into the desired shape.

The structured gel-filled cosmetic mask according to the technical solution of the present invention can be made into a facial mask, eye mask, nasal mask, lip mask, frontal mask or other cosmetic facial masks as needed.

The present invention adopts the above technical solution and fully considers and solves the following problems:
1. The active ingredients (the premade filling material) of the invention are integrally formed with the fixing skeleton, a part of which is stored in the cavity of the fixing skeleton and the rest of which is free to extend outward to form a free-flow gel layer. Such an integrated structure is very stable, overcoming the shortcomings such as unstable in structure, easy to break of jelly alone, gel-like masks, and also overcoming drawbacks such as unstable in structure and insufficient active ingredients to be carried of a mask in which a material is used as support layer and a gel layer is attached to the support layer (in this layered structure, two layers are not closely combined).
2. When the cavity has a selected shape, especially a honeycomb shape or regular hexagonal shape arranged in an array, the fixing skeleton can firmly combine the gel filling band and the free-flow gel layer together, to exhibit greatly improved distortion resistance and tensile strengthen, enhanced ability to carry active ingredients, and more uniform and smooth surface of the obtained products.
3. According to the experiments and tests, when the cavity has an area of no more than 9 mm², and/or the cavity has a diameter of 0.09 mm-1.0 mm, the mask is composed of polyester fibers, and/or the gel of the gel filling band has a viscosity of 150,000 to 500,000 mPa.s and a density of 0.5 to 1.5 g/cm³, an even better stability can be achieved.
4. When the free-flow gel layer coats the gel filling band on both sides, and the thickness ratio of the gel filling band to the free-flow gel layer is 1:2 to 1:10, a better elasticity and tensile strength are achieved, and better effects in stretching skin wrinkles are fulfilled.
5. For the product with the four formulations and their weight proportions as defined in the claims of the present invention, the components cooperate with each other to play better stability than other solutions, and the gel can be completely dissolved in water, so that the active ingredients can be fully released on the skin to achieve better cosmetic results.
6. The preparation method as described in the claims of the present invention provides a convenient process to prepare the structured gel-filled cosmetic mask of the present invention, which can meet the demand and effectively save the cost.

In comparison with the prior art, the technical solution of the invention has the advantages of providing a structured gel-filled cosmetic mask, in which architectural structure and method are incorporated into the cosmetic mask, employing a fixing skeleton, a gel filling band and a free-flow gel layer in an integrally formed structure that utilizes the cavity with a concave structure of the fixing skeleton, in combination with the cosmetic ingredients in gel form, to produce elasticity and stretching force, making the cosmetic mask more stable, bearing more active ingredients, and exhibiting stretch effect when applying on skin wrinkles, and thus it is economical and suitable for being widely used in the art.

### Brief Description of the Invention

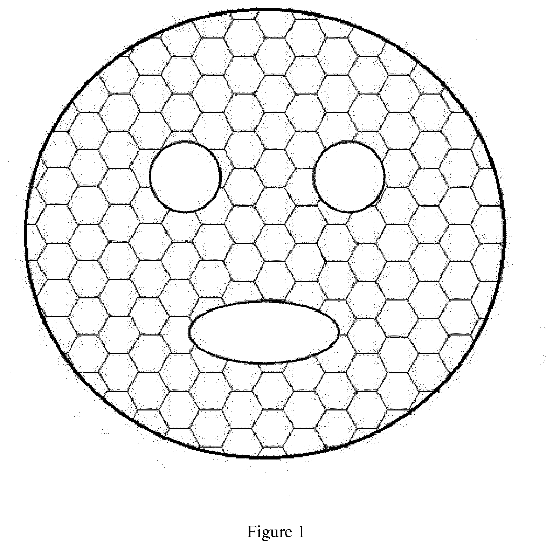

Figure 1 is an overall schematic view of a cosmetic mask of the present invention;

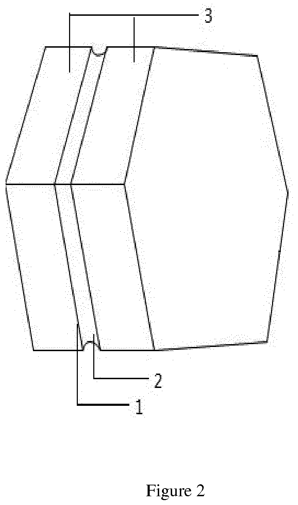

Figure 2 is a schematic structural view of a cavity unit of the present invention;

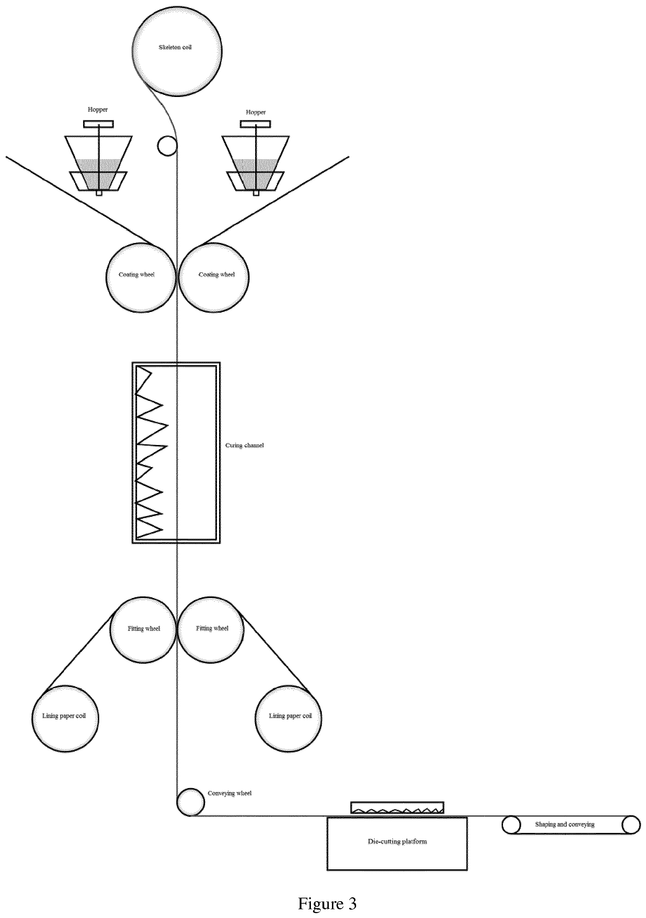

Figure 3 is a flow chart of the preparation of a cosmetic mask of the present invention.

### Embodiments

The present invention is further illustrated in conjunction with the drawings and examples.

### Example 1

A structured gel-filled cosmetic mask comprising a fixing skeleton 1, a gel filling band 2 and a free-flow gel layer 3 in an integrally formed structure, in which the fixing skeleton 1 comprising cavities with concave structure, the cavities of the fixing skeleton are in honeycomb shape, the cavities are arranged in an array, the cavities are filled with a premade filling material to form the gel filling band 2, and the gel of the gel filling band 2 extends outward to form the free-flow gel layer 3, each single cavity of the fixing skeleton 1 has an area of not exceeding 9 mm², the cavities have a skeleton diameter of 0.09 mm to 1.0 mm, which is made of polyester fiber, the gel of the gel filling band 2 has a viscosity of 150,000 to 500,000 mPa.s and a density of 0.5 to 1.5 g/cm³, the free-flow gel layer 3 coats both sides of the gel filling band 2, the gel filling band 2 and the free-flow gel layer 3 have a thickness ratio of between 1:2 and 1:10; the premade filling material iss of the following formula:

| | |
|---|---|
| Glycerol | 25% |
| Water | 66.05% |
| Sodium Polyacrylate NOVETAC P46N | 5.0% |
| Sodium Polyacrylate NOVETAC P55N | 0.5% |
| Dihydroxyaluminum aminoacetate | 0.15% |
| Hydrolyzed elastin | 1.0% |
| Sodium Hyaluronate | 0.5% |
| Vitamin E | 0.5% |
| VC polysaccharide | 0.3% |
| Paraben | 0.1% |
| Tartaric acid | 0.2% |
| Essence | 0.2% |
| Ethanol | 0.5% |

### Example 2

A structured gel-filled cosmetic mask comprising a fixing skeleton 1, a gel filling band 2 and a free-flow gel layer 3 in an integrally formed structure, in which the fixing skeleton 1 comprising cavities with concave structure, the cavities of the fixing skeleton are in honeycomb shape, the cavities are arranged in an array, the cavities are filled with a premade filling material to form the gel filling band 2, and the gel of the gel filling band 2 extends outward to form the free-flow gel layer 3, each single cavity of the fixing skeleton 1 has an area of not exceeding 9 mm², the cavities have a skeleton diameter of 0.09 mm, which iss made of polyester fiber, the gel of the gel filling band 2 has a viscosity of 150,000 mPa.s and a density of 0.5 g/cm³, the free-flow gel layer 3 coats both sides of the gel filling band 2, the gel filling band 2 and the free-flow gel layer 3 have a thickness ratio of 1:2; the premade filling material iss of the following formula:

| | |
|---|---|
| Glycerin | 23% |
| Water | 65.34% |
| Sodium Polyacrylate AP800 | 6.0% |
| Dihydroxyaluminum aminoacetate | 0.16% |
| Sodium Hyaluronate | 0.5% |
| Collagen | 1.0% |
| Aloe leaf extract | 1.0% |
| Arbutin | 0.5% |
| Vitamin C | 0.3% |
| Hydantoin DMDM | 0.2% |
| Tartaric acid | 0.2% |
| Essence | 0.2% |
| Ethanol | 0.6% |

### Example 3

A structured gel-filled cosmetic mask comprising a fixing skeleton 1, a gel filling band 2 and a free-flow gel layer 3 in an integrally formed structure, in which the fixing skeleton 1 comprising cavities with concave structure, the cavities of the fixing skeleton are in honeycomb shape, the cavities are arranged in an array, the cavities are filled with a premade filling material to form the gel filling band 2, and the gel of the gel filling band 2 extends outward to form the free-flow gel layer 3, each single cavity of the fixing skeleton 1 has a skeleton diameter of 1.0 mm, which is made of polyester fiber, the gel of the gel filling band 2 has a viscosity of 500,000 mPa.s and a density of 1.5 g/cm³, the free-flow gel layer 3 coats both sides of the gel filling band 2, the gel filling band 2 and the free-flow gel layer 3 have a thickness ratio of 1:10; the premade filling material is of the following formula:

| | |
|---|---|
| Water | 46.15% |
| Glycerin | 44.9% |
| Carboxymethyl cellulose | 3.0% |
| Lubrajel | 0.8% |
| Xanthan gum | 1.0% |
| Pectin | 1.0% |
| Disodium EDTA | 0.5% |
| Sodium Hyaluronate | 1.0% |
| Aloe vera Leaf Extract | 1.0% |
| Aloperine | 0.3% |
| Oat peptide | 0.2% |
| Peppermint | 0.05% |
| Essence | 0.1% |

### Example 4

A structured gel-filled cosmetic mask comprising a fixing skeleton 1, a gel filling band 2 and a free-flow gel layer 3 in an integrally formed structure, in which the fixing skeleton 1 comprising cavities with concave structure, the cavities of the fixing skeleton are in honeycomb shape, the cavities were arranged in an array, the cavities are filled with a premade filling material to form the gel filling band 2, and the gel of the gel filling band 2 extends outward to form the free-flow gel layer 3, each single cavity of the fixing skeleton 1 has a skeleton diameter of 0.59 mm, which is made of polyester fiber, the gel of the gel filling band 2 has a viscosity of 320,000 mPa.s and a density of 1 g/cm³, the free-flow gel layer 3 coats both sides of the gel filling band 2, the gel filling band 2 and the free-flow gel layer 3 have a thickness ratio of 1:6; the premade filling material is of the following formula:

| | |
|---|---|
| Water | 24% |
| Glycerin | 64.5% |
| Hydroxyethyl cellulose | 3.0% |
| Konjac gum | 3.0% |
| Carbopol resin | 0.8% |
| Disodium EDTA | 0.5% |
| Arbutin | 2.0% |
| Sodium Hyaluronate | 0.5% |
| Egg White-Sodium | 0.1% |
| Collagen | 1.0% |
| Essence | 0.1% |
| Sodium hydroxide | 0.5% |

### Preparation method example

The structured gel-filled cosmetic mask according to the technical solution of the present invention was prepared by the following steps:
Step 1), preparation of the premade filling material: weighing ingredients, loading the ingredients into a mixer, mixing and emulsifying to form the premade filing material;
Step 2), injection: heating the premade filing material, and pouring or pumping the premade filling material into hoppers on both sides of a special coater; passing the fixing skeleton through two middle coating wheels between the hoppers on both sides; starting the coating wheels to rotate, injecting the gel downflowing from the hoppers on both sides into the fixing skeleton by the coating wheels to form an integrally formed matrix membrane;
Step 3), formation of mask: conveying the integrally formed matrix membrane as prepared in Step 2) via a conveying wheel to a gel curing channel, accelerating the curing of the matrix membrane in the curing channel by a high temperature drying method or a cooling method, then conveying to a die-cutting platform, selecting a die according to the shape of the product to be produced, and cutting the membrane into the desired shape.

The foregoing is a detailed description of the present invention in conjunction with specific examples, and it should not be considered that the specific embodiment of the present invention is limited to these descriptions. Those skilled in the art to which the present invention pertains may also make some simple deductions or replacements without departing from the concept of the present invention, which all fall into the scope of protection of the present invention.

## Claims

1. A structured gel-filled cosmetic mask, comprising a fixing skeleton, a gel filling band and a free-flow gel layer in an integrally formed structure, wherein the fixing skeleton comprises a cavity of a concave structure, the cavity is filled with a premade filling material to form the gel filling band, and the gel in the gel filling band extends outward to form the free-flow gel layer.

2. The structured gel-filled cosmetic mask according to claim 1, **characterized in that** the cavity of the fixing skeleton is triangular, square, polygonal or irregular in shape, and a plurality of cavities are arranged in an array.

3. The structured gel-filled cosmetic mask according to claim 1, **characterized in that** the shape of the cavity of the fixing skeleton is a honeycomb or a regular hexagon, and a plurality of cavities are arranged in an array.

4. The structured gel-filled cosmetic mask according to claim 1, **characterized in that** a single cavity of the fixing skeleton has an area of no more than 9 mm².

5. The structured gel-filled cosmetic mask according to claim 3, **characterized in that** the cavity of the fixing skeleton has a diameter of 0.09 mm to 1.0 mm, and is made of polyester fiber.

6. The structured gel-filled cosmetic mask according to claim 1, **characterized in that** the gel in the gel-filled band has a viscosity of 150,000 to 500,000 mPa·s and a density of 0.5 to 1.5 g/cm³.

7. The structured gel-filled cosmetic mask according to claim 1, **characterized in that** the free-flow gel layer coats both sides of the gel-filled band, and the thickness ratio of the gel-filled band to the free-flow gel layer is 1:2 to 1:10.

8. A structured gel-filled cosmetic mask according to claim 1, **characterized in that**, the premade filling material is one selected from the following four formulas:
**Formula 1:**
| | |
|---|---|
| Glycerol | 25% |
| Water | 66.05% |
| Sodium Polyacrylate NOVETAC P46N | 5.0% |
| Sodium Polyacrylate NOVETAC P55N | 0.5% |
| Dihydroxyaluminum aminoacetate | 0.15% |
| Hydrolyzed elastin | 1.0% |
| Sodium Hyaluronate | 0.5% |
| Vitamin E | 0.5% |
| VC polysaccharide | 0.3% |
| Paraben | 0.1% |
| Tartaric acid | 0.2% |
| Essence | 0.2% |
| Ethanol | 0.5% |
**Formula 2**
| | |
|---|---|
| Glycerin | 23% |
| Water | 65.34% |
| Sodium Polyacrylate AP800 | 6.0% |
| Dihydroxyaluminum aminoacetate | 0.16% |
| Sodium Hyaluronate | 0.5% |
| Collagen | 1.0% |
| Aloe leaf extract | 1.0% |
| Arbutin | 0.5% |
| Vitamin C | 0.3% |
| Hydantoin DMDM | 0.2% |
| Tartaric acid | 0.2% |
| Essence | 0.2% |
| Ethanol | 0.6% |
**Formula 3**
| | |
|---|---|
| Water | 46.15% |
| Glycerin | 44.9% |
| Carboxymethyl cellulose | 3.0% |
| Lubrajel | 0.8% |
| Xanthan gum | 1.0% |
| Pectin | 1.0% |
| Disodium EDTA | 0.5% |
| Sodium Hyaluronate | 1.0% |
| Aloe vera Leaf Extract | 1.0% |
| Aloperine | 0.3% |
| Oat peptide | 0.2% |
| Peppermint | 0.05% |
| Essence | 0.1% |
**Formula 4**
| | |
|---|---|
| Water | 24% |
| Glycerin | 64.5% |
| Hydroxyethyl cellulose | 3.0% |
| Konjac gum | 3.0% |
| Carbopol resin | 0.8% |
| Disodium EDTA | 0.5% |
| Arbutin | 2.0% |
| Sodium Hyaluronate | 0.5% |
| Egg White-Sodium | 0.1% |
| Collagen | 1.0% |
| Essence | 0.1% |
| Sodium hydroxide | 0.5% |

9. The structured gel-filled cosmetic mask according to claim 1, **characterized in** being prepared by the following steps:
Step 1), preparation of the premade filling material: weighing ingredients, loading the ingredients into a mixer, mixing and emulsifying to form the premade filing material;
Step 2), injection: pouring or pumping the premade filling material into hoppers on both sides of a coater; passing the fixing skeleton through two middle coating wheels between the hoppers on both sides; starting the coating wheels to rotate, injecting the gel downflowing from the hoppers on both sides into the fixing skeleton by the coating wheels to form an integrally formed matrix membrane;
Step 3), formation of mask: conveying the integrally formed matrix membrane as prepared in Step 2) via a conveying wheel to a gel curing channel, accelerating the curing of the matrix membrane in the curing channel by a high temperature drying method or a cooling method, then conveying to a die-cutting platform, selecting a die according to the shape of the product to be produced, and cutting the membrane into the desired shape.

10. The structured gel-filled cosmetic mask according to claim 1, **characterized in that** the cosmetic mask is made into a facial mask, eye mask, nasal mask, lip mask, or frontal mask as needed.

## Patentansprüche

1. Strukturierte, Gel-gefüllte kosmetische Maske, aufweisend ein Befestigungsgrundgerüst, ein Gelfüllband und eine freifließende Gelschicht in einer integral ausgebildeten Struktur, wobei das Befestigungsgrundgerüst eine Kavität mit konkavem Aufbau aufweist, die Kavität mit einem zuvor hergestellten Füllmaterial zum Bilden des Gelfüllbands gefüllt wird, und sich das Gel in dem Gelfüllband nach außen erstreckt, um die freifließende Gelschicht zu bilden.

2. Strukturierte, Gel-gefüllte kosmetische Maske nach Anspruch 1, **dadurch gekennzeichnet, dass** das Befestigungsgrundgerüst dreieckig, viereckig, polygonal oder formunregelmäßig ist, und eine Vielzahl von Kavitäten in einem Array angeordnet sind.

3. Strukturierte, Gel-gefüllte kosmetische Maske nach Anspruch 1, **dadurch gekennzeichnet, dass** die Form der Kavität des Befestigungsgrundgerüsts eine Bienenwabe oder ein regelmäßiges Sechseck ist, und eine Vielzahl von Kavitäten in einem Array angeordnet sind.

4. Strukturierte, Gel-gefüllte kosmetische Maske nach Anspruch 1, **dadurch gekennzeichnet, dass** eine einzelne Kavität des Befestigungsgrundgerüsts einen Flächeninhalt von nicht mehr als 9 mm² hat.

5. Strukturierte, Gel-gefüllte kosmetische Maske nach Anspruch 3, **dadurch gekennzeichnet, dass** die Kavität des Befestigungsgrundgerüsts einen Durchmesser von 0,09 mm bis 1,0 mm hat und aus Polyesterfasern hergestellt ist.

6. Strukturierte, Gel-gefüllte kosmetische Maske nach Anspruch 1, **dadurch gekennzeichnet, dass** das Gel in dem Gel-gefüllten Band eine Viskosität von 150.000 bis 500.000 mPa·s und eine Dichte von 0,5 bis 1,5 g/cm³ hat.

7. Strukturierte, Gel-gefüllte kosmetische Maske nach Anspruch 1, **dadurch gekennzeichnet, dass** die freifließende Gelschicht beide Seiten des Gel-gefüllten Bands bedeckt und das Dickenverhältnis des Gel-gefüllten Bands zur freifließenden Gelschicht 1:2 bis 1:10 beträgt.

8. Strukturierte, Gel-gefüllte kosmetische Maske nach Anspruch 1, **dadurch gekennzeichnet, dass** das vorgefertigte Füllmaterial eines ist, das aus den nachstehenden vier Formel ausgewählt ist:
**Formel 1:**
| | |
|---|---|
| Glycerol | 25 % |
| Wasser | 66,05 % |
| Natriumpolyacrylat NOVETAC P46N | 5,0 % |
| Natriumpolyacrylat NOVETAC P55N | 0,5 % |
| Dihydroxyaluminiumaminoacetat | 0,15 % |
| Hydrolysiertes Elastin | 1,0 % |
| Natriumhyaluronat | 0,5 % |
| Vitamin E | 0,5 % |
| VC-Polysaccharid | 0,3 % |
| Paraben | 0,1 % |
| Weinsteinsäure | 0,2 % |
| Extrakt | 0,2 % |
| Ethanol | 0,5 % |
**Formel 2 :**
| | |
|---|---|
| Glycerin | 23 % |
| Wasser | 65,34 % |
| Natriumpolyacrylat AP800 | 6,0 % |
| Dihydroxyaluminiumaminoacetat | 0,16 % |
| Natriumhyaluronat | 0,5 % |
| Kollagen | 1,0 % |
| Aloe-Vera-Extrakt | 1,0 % |
| Arbutin | 0,5 % |
| Vitamin C | 0,3 % |
| Hydantonin DMDM | 0,2 % |
| Tartarsäure | 0,2 % |
| Extrakt | 0,2 % |
| Ethanol | 0,6 % |
**Formel 3:**
| | |
|---|---|
| Wasser | 46,15 % |
| Glycerin | 44,90 % |
| Carboxymethylcellulose | 3,0 % |
| Lubrajel | 0,8 % |
| Xanthangummi | 1,0 % |
| Pektin | 1,0 % |
| Dinatrium-EDTA | 0,5 % |
| Natriumhyaluronat | 1,0 % |
| Aloe-Vera-Blattextrakt | 1,0 % |
| Aloperin | 0,3 % |
| Haferpeptid | 0,2 % |
| Pfefferminze | 0,05 % |
| Extrakt | 0,1 % |
**Formel 4:**
| | |
|---|---|
| Wasser | 24 % |
| Glyerin | 64,5 % |
| Hydroxyethylcellulose | 3,0 % |
| Konjakgummi | 3,0 % |
| Carbopolharz | 0,8 % |
| Dinatrium-EDTA | 0,5 % |
| Arbutin | 2,0 % |
| Natriumhyaluronat | 0,5 % |
| Eiweißnatrium | 0,1 % |
| Kollagen | 1,0 % |
| Extrakt | 0,1 % |
| Natriumhydroxid | 0,5 % |

9. Strukturierte, Gel-gefüllte kosmetische Maske nach Anspruch 1, **dadurch gekennzeichnet, dass** sie mit den nachfolgenden Schritten hergestellt wird:
Schritt 1), Herstellen des vorgefertigten Füllmaterials: Einwiegen der Inhaltsstoffe, Einfüllen der Inhaltsstoffe in einen Mischer, Mischen und Emulgieren, um das vorgefertigte Füllmaterial zu bilden;
Schritt 2) Injektion: Gießen oder Pumpen des vorgefertigten Füllmaterials in Fülltrichter an beiden Seiten einer Beschichtungseinrichtung; Hindurchleiten des Befestigungsgrundgerüsts durch zwei mittlere Beschichtungsräder zwischen den Fülltrichtern auf beiden Seiten; Beginn der Rotation der Räder; Injizieren des Gels, das aus den Fülltrichtern nach unten strömt, an beiden Seiten in das Befestigungsgrundgerüst, durch die Beschichtungsräder, um eine integral ausgebildete Matrixmembran zu bilden;
Schritt 3) Bildung der Maske: Transportieren der wie in Schritt 2) hergestellten integral ausgebildeten Matrixmembran über ein Förderrad zu einem Gelhärtungskanal, Beschleunigen der Aushärtung der Matrixmembran in dem Härtungskanal durch ein Hochtemperaturtrocknungsverfahren oder ein Kühlverfahren, dann Fördern an eine Stanzplattform, Auswählen eines Schneidwerkzeugs gemäß der Form des herzustellenden Erzeugnisses, und Schneiden der Membran in die gewünschte Form.

10. Strukturierte, Gel-gefüllte kosmetische Maske nach Anspruch 1, **dadurch gekennzeichnet, dass** die kosmetische Maske je nach Bedarf zu einer Gesichtsmaske, Augenmaske, Nasenmaske, Lippenmaske oder Stirnmaske gemacht wird.

## Revendications

1. Masque cosmétique rempli de gel, structuré, comprenant un squelette de fixation, une bande de remplissage de gel et une couche de gel à écoulement libre dans une structure formée d'un seul tenant, le squelette de fixation comprenant une cavité à structure concave, la cavité étant remplie d'un matériau de remplissage pré-préparé afin de former la bande de remplissage de gel, et le gel dans la bande de remplissage de gel s'étendant vers l'extérieur afin de former la couche de gel à écoulement libre.

2. Le masque cosmétique rempli de gel, structuré, selon la revendication 1, **caractérisé en ce que** la cavité du squelette de fixation prend une forme triangulaire, carrée, polygonale ou irrégulière, et plusieurs cavités sont agencées dans une matrice.

3. Le masque cosmétique rempli de gel, structuré, selon la revendication 1, **caractérisé en ce que** la forme de la cavité du squelette de fixation est un nid d'abeilles ou un hexagone régulier, et plusieurs cavités sont agencées dans une matrice.

4. Le masque cosmétique rempli de gel, structuré, selon la revendication 1, **caractérisé en ce qu'**une seule cavité du squelette de fixation présente une superficie inférieure ou égale à 9 mm².

5. Le masque cosmétique rempli de gel, structuré, selon la revendication 3, **caractérisé en ce que** la cavité du squelette de fixation présente un diamètre compris entre 0,09 mm et 1,0 mm et elle est faite en fibre de polyester.

6. Le masque cosmétique rempli de gel, structuré, selon la revendication 1, **caractérisé en ce que** le gel dans la bande rempli de gel présente une viscosité comprise entre 150 000 et 500 000 mPa·s et une densité comprise entre 0,5 et 1,5 g/cm³.

7. Le masque cosmétique rempli de gel, structuré, selon la revendication 1, **caractérisé en ce que** la couche de gel à écoulement libre recouvre les deux côtés de la bande rempli de gel, et le rapport d'épaisseur de la bande rempli de gel à la couche de gel à écoulement libre est compris entre 1:2 et 1:10.

8. Un masque cosmétique rempli de gel, structuré, selon la revendication 1, **caractérisé en ce que** le matériau de remplissage pré-préparé comporte une formule choisie parmi les quatre formules suivantes :
**Formule 1**
| | |
|---|---|
| Glycérol | 25 % |
| Eau | 66,05 % |
| Polyacrylate de sodium NOVETAC P46N | 5,0 % |
| Polyacrylate de sodium NOVETAC P55N | 0,5 % |
| Aminoacétate de dihydroxyaluminium | 0,15 % |
| Élastine hydrolysée | 1,0 % |
| Hyaluronate de sodium | 0,5 % |
| Vitamine E | 0,5 % |
| Polysaccharide VC | 0,3 % |
| Parabène | 0,1 % |
| Acide tartrique | 0,2 % |
| Essence | 0,2 % |
| Éthanol | 0,5 % |
**Formule 2**
| | |
|---|---|
| Glycérine | 23 % |
| Eau | 65,34 % |
| Polyacrylate de sodium AP800 | 6,0 % |
| Aminoacétate de dihydroxyaluminium | 0,16 % |
| Hyaluronate de sodium | 0,5 % |
| Collagène | 1,0 % |
| Extrait de feuille d'aloès | 1,0 % |
| Arbutine | 0,5 % |
| Vitamine C | 0,3 % |
| Hydantoïne DMDM | 0,2 % |
| Acide tartrique | 0,2 % |
| Essence | 0,2 % |
| Éthanol | 0,6 % |
**Formule 3**
| | |
|---|---|
| Eau | 46,15 % |
| Glycérine | 44,9 % |
| Carboxyméthyl cellulose | 3,0 % |
| Lubrajel | 0,8 % |
| Gomme xanthane | 1,0 % |
| Pectine | 1,0 % |
| EDTA de disodium | 0,5 % |
| Hyaluronate de sodium | 1,0 % |
| Extrait de feuille d'aloès officinal | 1,0 % |
| Alopérine | 0,3 % |
| Peptide d'avoine | 0,2 % |
| Menthe poivrée | 0,05 % |
| Essence | 0,1 % |
**Formule 4**
| | |
|---|---|
| Eau | 24 % |
| Glycérine | 64,5 % |
| Hydroxyéthyl cellulose | 3,0 % |
| Gomme Konjac | 3,0 % |
| Résine Carbopol | 0,8 % |
| EDTA de disodium | 0,5 % |
| Arbutine | 2,0 % |
| Hyaluronate de sodium | 0,5 % |
| Blanc d'œuf-Sodium | 0,1 % |
| Collagène | 1,0 % |
| Essence | 0,1 % |
| Hydroxyde de sodium | 0,5 % |

9. Le masque cosmétique rempli de gel, structuré, selon la revendication 1, **caractérisé en ce qu'**il est préparé au moyen des étapes suivantes :
Étape 1), préparation du matériau de remplissage pré-préparé : peser les ingrédients, charger les ingrédients dans un mélangeur, mélanger et émulsifier afin de former le matériau de remplissage pré-préparé ;
Étape 2), injection : verser ou pomper le matériau de remplissage pré-préparé dans des trémies des deux côtés d'une machine à recouvrir ; passer le squelette de fixation à travers deux roues de recouvrement intermédiaires entre les trémies des deux côtés ; démarrer la rotation des roues de recouvrement ; injecter le gel coulant vers le bas à partir des trémies des deux côtés dans le squelette de fixation au moyen des roues de recouvrement, afin de former une membrane à matrice formée d'un seul tenant ;
Étape 3), formation du masque : transporter la membrane à matrice formé d'un seul tenant, préparée dans l'Étape 2), par l'intermédiaire d'une roue de transport vers un canal de traitement de gel, accélérer le traitement de la membrane à matrice dans le canal de traitement au moyen d'un procédé de séchage à haute température ou d'un procédé de refroidissement, puis la transporter vers une plateforme de découpage à l'emporte-pièce, sélectionner un emporte-pièce en fonction de la forme du produit à produire, et découper la membrane à la forme souhaitée.

10. Le masque cosmétique rempli de gel, structuré, selon la revendication 1, **caractérisé en ce que** le masque cosmétique prend une forme de masque facial, de masque oculaire, de masque nasal, de masque pour les lèvres ou de masque pour le front, selon les besoins.
